# FASCICULE DE BREVET EUROPEEN

(11) **EP 1 933 635 B1**
(45) Date de publication et mention de la délivrance du brevet: **11.02.2009**
(21) Numéro de dépôt: 06777869.6
(22) Date de dépôt: 20.07.2006
(51) Int. Cl.: A23K 1/00, A23K 1/18

(54) **FACTEUR D'APPETENCE FERMENTE ET DEPOURVU DE PROTEINES ANIMALES POUR ANIMAUX**
FERMENTIERTE APPETENZFAKTOREN OHNE TIERISCHE EIWEISSE FÜR TIERE
FERMENTED APPETENCE FACTORS WITHOUT ANIMAL PROTEINS FOR ANIMALS

(30) Priorité: 09.08.2005 FR 0508451
(43) Date de publication de la demande: 25.06.2008
(73) Titulaire: Specialites Pet Food, 75008 Paris (FR)
(72) Inventeur: DE RATULD, Aurélie, F-56880 Ploeren (FR); THIRIOT, Rémi, F-56460 Lizio (FR); GUILLER, Isabelle, F-56370 Le Tour du Parc (FR)
(74) Mandataire: Ahner, Francis
(86) Numéro de dépôt international: PCT/EP2006/064474
(87) Numéro de publication internationale: WO 2007/017356

(56) Documents cités:
- GB-A- 1 584 539
- GB-A- 2 385 767
- US-A- 4 039 687
- US-A- 4 800 093
- US-A- 5 000 964

## Description

La présente invention se rapporte au domaine des facteurs d'appétence pour l'alimentation animale.

Plus précisément, la présente invention concerne un facteur d'appétence pour l'alimentation animale, obtenu par fermentation à l'aide d'une levure et qui comprend au moins, en l'absence de constituants protéiques d'origine animale:
- humidité (H) : de 80 à 96% en poids environ ;
- fraction protéique (FP) : de 1,5 à 10,0% en poids environ ;
- matières grasses (MG) : jusqu'à 10% en poids environ ;
- sucres (S) : de 0,20 à 2,0% en poids environ ;
- cendres (C) : de 0,3 à 8,0% en poids environ.

On recherche, à travers l'alimentation donnée aux animaux, notamment aux animaux de compagnie, à favoriser et préserver leur santé, leur bien-être et leur équilibre. Toutefois, même les aliments les plus bénéfiques d'un point de vue nutritionnel sont sans intérêt si l'animal les rejette ou refuse de les manger, ou encore si les rations prises par l'animal restent faibles parce que la nourriture n'est pas suffisamment à son goût.

Aussi, afin de faciliter la prise de nourriture par les animaux, et plus particulièrement par les animaux domestiques tels que les chiens et les chats qui se révèlent bien souvent difficiles, on a très tôt cherché à améliorer l'appétence des aliments, tout en préservant leurs qualités nutritionnelles.

Typiquement, la viande et le poisson représentent les principales sources de protéines dans la plupart des aliments pour animaux. En effet, la nature et la quantité des acides aminés apportés par les dérivés animaux (viande, os, poisson) confèrent aux aliments les qualités nutritionnelles attendues. En outre, ces derivés ont pour effet d'augmenter l'appétence des aliments qui les contiennent.

Toutefois, la présence de constituants d'origine animale dans les aliments augmente de manière considérable leur coût de revient.

C'est pourquoi, les recherches se sont très vite orientées vers la mise au point d'aliments et ingrédients alimentaires nutritionnellement équilibrés, aussi appétissants que les aliments et ingrédients traditionnels à base de matière animale (par exemple, à base de foie), mais de préparation moins coûteuse. En effet, l'industrie de l'alimentation animale a très vite mesuré l'intérêt de trouver des sources de protéines stables, nutritionnellement satisfaisantes et, surtout, économiques, afin de les substituer à la viande ou au poisson lors de la préparation d'aliments et ingrédients alimentaires pour animaux.

Dans ce contexte, le brevet américain US 4 039 687 (au nom de Weyn) propose de substituer tout ou partie des protéines animales présentes dans les aliments par des protéines synthétiques obtenues par fermentation de microorganismes, e.g., de bactéries ou de levures. Les aliments ainsi obtenus présentent des propriétés d'appétence satisfaisantes et peuvent être fabriqués à moindre coût par rapport à des aliments à base de protéines naturelles d'origine animale ou végétale. En pratique, on incorpore aux aliments, en tant que source de protéines, la biomasse obtenue après fermentation des microorganismes sur des hydrocarbures.

Le brevet US 4 800 093 (au nom de Hogan et al.) décrit un produit alimentaire pour animaux à taux d'humidité élevé, dans lequel une proportion de la matière première carnée est remplacée par la biomasse obtenue après fermentation de champignons filamenteux sur des substrats bon marché tels que le petit-lait de soja.

La demande de brevet britannique GB 2 385 767 (au nom de Norferm DA) s'est intéressée aux cultures de bactéries méthanotrophes comme facteurs d'appétence. La biomasse utilisée est obtenue par fermentation d'une bactérie du type *Methylococcus capsulatus* sur des hydrocarbures ou du gaz naturel.

Ainsi, d'après l'état de la technique, il est possible de substituer aux protéines animales, sans préjudice pour l'appétence et les qualités nutritionnelles des aliments préparés, des protéines synthétisées par des microorganismes, que l'on ajoute aux aliments sous la forme d'une biomasse obtenue après fermentation.

Toutefois, l'ajout dans l'alimentation animale d'une biomasse comme facteur d'appétence n'est pas sans inconvénients, notamment parce qu'un tel ingrédient impose nécessairement que soient mises en oeuvre (i) une étape d'inactivation efficace de la biomasse, afin de prévenir toute modification intempestive des propriétés organoleptiques de l'aliment auquel il sera ajouté (due, par exemple, à une fermentation ou à une croissance indésirable des microorganismes) ; et (ii) une étape de séparation afin d'éliminer le milieu de fermentation. De plus, un tel ingrédient a bien souvent tendance à modifier l'aspect (e.g., la couleur) des aliments obtenus.

Il existe donc un besoin pour des facteurs d'appétence pour animaux, qui soient à la fois : (i) équilibrés quant aux apports nutritionnels et caloriques ; (ii) au moins aussi efficaces que les facteurs conventionnels à base de dérivés animaux ; (iii) faciles à préparer, à partir de matières premières aisément accessibles et économiques ; (iv) stables dans le temps ; et (v) aussi neutres que possible au niveau de leurs propriétés physiques (couleur, viscosité, etc...), afin d'une part, de ne pas modifier l'aspect et la texture des aliments auxquels ils sont ajoutés et, d'autre part, de ne pas poser de difficultés d'utilisation particulières (par exemple, en cas de pulvérisation sur les aliments).

Dans le cadre de la présente invention, la Demanderesse propose un facteur d'appétence pour animaux qui permet de combler, de manière aussi satisfaisante que surprenante, le besoin existant dans le domaine. Comme l'illustrent la description détaillée et les exemples ci-dessous, (1) le facteur d'appétence selon l'invention est nutritionnellement équilibré ; (2) il s'agit d'un produit végétarien et hypoallergénique ; (3) il est plus efficace que les facteurs d'appétence traditionnels à base de protéines d'origine animale ; (4) il est obtenu aisément par fermentation de microorganismes, de préférence de levures ; (5) de manière surprenante, on utilise le milieu de fermentation lui-même en tant que facteur d'appétence, ce qui permet de s'affranchir d'une des étapes requises pour produire les facteurs d'appétence de l'état de la technique à base de biomasse car, selon l'invention, soit l'on utilise le milieu de fermentation seul - il suffit alors d'éliminer la biomasse et l'étape d'inactivation devient inutile -, soit l'on utilise le milieu de fermentation en présence de la biomasse - il convient alors simplement d'inactiver efficacement la biomasse ; (6) ses propriétés physiques sont globalement neutres : c'est un liquide aqueux, de couleur claire, qui se fond facilement dans une masse ; (7) il est facile à utiliser, notamment par application, pulvérisation, incorporation, imprégnation.

Au sens de l'invention, un « facteur d'appétence » (ou, de manière équivalente, un « agent d'appétence », un facteur « appétent » ou « appétissant ») pour animaux est un facteur dont les propriétés organoleptiques (parfum, goût, aspect, texture, etc.) sont telles qu'un animal va éprouver le désir de l'absorber. Dans le cadre de la présente invention, un facteur d'appétence est le plus souvent un ingrédient alimentaire (ou additif) que l'on ajoutera à l'alimentation animale. Mais, par extension, ce peut également être un aliment, voire une boisson, contenant un tel ingrédient.

Ainsi, un premier aspect de la présente invention concerne un facteur d'appétence fermenté destiné à être ajouté à l'alimentation animale, caractérisé en ce qu'il comprend le milieu de fermentation obtenu après culture d'au moins une levure, ledit milieu de fermentation contenant au moins :
- humidité (H) : de 80 à 96% en poids environ ;
- fraction protéique (FP) : de 1,5 à 10,0% en poids environ ;
- matières grasses (MG) : jusqu'à 10% en poids environ ;
- sucres (S) : de 0,20 à 2,0% en poids environ ; et
- cendres (C) : de 0,3 à 8,0% en poids environ,
où ladite fraction protéique FP est dépourvue de constituants d'origine animale.

Par exemple, des facteurs d'appétence particuliers comprendront au moins, en l'absence de constituants protéiques d'origine animale :
- humidité (H) : de 85 à 96 % en poids environ ;
- fraction protéique (FP) : de 1,5 à 10,0% en poids environ ;
- matières grasses (MG) : jusqu'à 1% en poids environ ;
- sucres (S) : de 0,20 à 2,0% en poids environ ; et
- cendres (C) : de 0,3 à 8,0% en poids environ ;
ou
- humidité (H) : de 90 à 96 % en poids environ ;
- fraction protéique (FP) : de 2,5 à 10,0% en poids environ ;
- matières grasses (MG) : jusqu'à 5% en poids environ ;
- sucres (S) : de 0,30 à 0,75% en poids environ ; et
- cendres (C) : de 0,5 à 0,7% en poids environ ;
ou encore
- humidité (H) : de 90 à 96 % en poids environ ;
- fraction protéique (FP) : de 2,5 à 10,0% en poids environ ;
- matières grasses (MG) : jusqu'à 1% en poids environ
- sucres (S) : de 0,30 à 0,75% en poids environ ; et
- cendres (C) : de 0,5 à 0,7% en poids environ.

Le terme « cendres » désigne ici la matière minérale après calcination du produit.

Par « matières grasses », on entend ici n'importe quel type de matières grasses adapté à l'alimentation animale et susceptible d'être métabolisé (c'est-à-dire, susceptible d'être consommé ou produit) par le microorganisme responsable de la fermentation à l'origine du facteur d'appétence. Il peut s'agir de matières grasses d'origine animale ou végétale ou d'une combinaison de celles-ci. L'homme du métier saura quel(s) type(s) de matières grasses utiliser en fonction de ses connaissances générales et des applications du facteur d'appétence envisagées.

Les « constituants de la fraction protéique FP » peuvent être des protéines, des peptides, des acides aminés, et des combinaisons de ceux-ci. Les termes « protéines » et « peptides » pourront parfois être utilisés indifféremment dans ce qui suit, étant entendu que l'homme du métier est capable de déterminer le sens exact à donner à l'un ou l'autre de ces termes d'après le contexte et à la lumière de ses connaissances générales. En outre, les expressions « constituants protéiques » et « constituants de la fraction protéique FP » sont ici équivalentes.

Selon un mode de réalisation, la fraction protéique FP comprend au moins 90% en poids environ d'un ou plusieurs acides aminés choisis parmi l'alanine, la thréonine, la lysine et les acides aminés soufrés. En particulier, la fraction protéique FP comprend au moins 95%, de préférence au moins 98%, de préférence encore au moins 99% en poids environ du ou desdits acides aminés.

De préférence, ce ou ces acides aminés sont choisis parmi les acides aminés soufrés, tels que la méthionine et la cystéine. On préférera utiliser la méthionine.

Selon un autre mode de réalisation, un facteur d'appétence conforme à l'invention présente un pH allant de 1,6 à 3,5 environ, étant entendu qu'un pH allant de 2,6 à 3,2 environ est préféré.

Un facteur d'appétence selon l'invention est « fermenté », c'est-à-dire qu'il est obtenu par fermentation à l'aide d'au moins un microorganisme, qui est, dans le cadre de l'invention, une levure. De préférence, la levure est choisie parmi *Yarrowia sp., Saccharomyces sp., Candida sp., Kluyveromyces sp., Pichia sp., Debaryomyces sp., Zygosaccharomyces sp.* De préférence encore, la levure est choisie parmi *Yarrowia lipolytica, Saccharomyces cerevisiae, Candida versalitis, Kluyveromyces lactis, Kluyveromyces fragilis, Pichia pastoris, Debaryomyces harsenii, Zygosaccharomyces rouxii.* De manière préférée entre toutes, on utilise la levure *Yarrowia lipolytica.*

Comme indiqué ci-dessus, un facteur d'appétence conforme à l'invention comprend le milieu de fermentation obtenu après culture de ladite levure. Eventuellement, le milieu de fermentation est utilisé comme agent d'appétence après avoir été débarrassé de la biomasse.

Selon encore un autre mode de réalisation, le facteur d'appétence se présente sous une forme liquide prête à l'emploi. Alternativement, il peut servir de produit intermédiaire dans la préparation d'un facteur d'appétence fermenté concentré. Dans ce cas, le facteur d'appétence concentré, à taux d'humidité réduit, peut se présenter sous une forme liquide ou sous une forme déshydratée. Par « concentré », l'on entend désigner un facteur d'appétence qui est obtenu par concentration d'un facteur d'appétence présentant les teneurs en H, FP, MG, S et C décrites supra. Il est bien évident que le facteur d'appétence concentré présente, du fait même de la concentration, des teneurs en FP, MG, S et C supérieures à celles spécifiées ci-dessus, tout en ayant un taux d'humidité plus faible.

Selon un mode de réalisation, on applique un facteur d'appétence de l'invention à l'alimentation animale. Alternativement, le facteur d'appétence est incorporé dans l'alimentation animale. De manière générale, on parle ici d' « ajout » ou d' « utilisation » d'un facteur d'appétence. Par « ajouter » ou « utiliser » un facteur d'appétence, on englobe la notion d' « appliquer le facteur à », c'est-à-dire l'ajouter en surface, par exemple par pulvérisation ou enrobage, et la notion d' « incorporer le facteur dans », c'est-à-dire l'ajouter dans la masse, par exemple par imprégnation ou mélange.

Par « alimentation animale », on désigne ici un ou plusieurs aliments pour animaux. Il peut s'agir de matières solides (e.g., croquettes), liquides (e.g., bouillons, boissons), ou de produits intermédiaires plus ou moins humides (e.g., soupes, bouillies, boulettes, pâtés, etc.). En fonction du contexte, il pourra également s'agir d'une ration alimentaire ou d'un repas, faisant alors référence à l'ensemble des aliments (sans les différencier) pris au cours d'un repas, voire sur une période de temps telle qu'une journée, ou plus.

Les termes « pour animaux » ou « animal, -e, -aux, -ales » doivent être pris dans leur sens le plus large, comme visant des animaux de tous les types. De préférence, les facteurs d'appétence selon l'invention, ou l'alimentation animale à laquelle ils sont ajoutés, sont destinés aux animaux de compagnie tels que les chiens et les chats, avec une préférence toute particulière pour les chiens. Toutefois, l'objet de l'invention s'adresse à n'importe quel animal susceptible d'être domestiqué ou apprivoisé ou élevé, par exemple, des oiseaux, des lapins, des rongeurs, des poissons, etc. Il peut également s'agir d'animaux d'élevage tels que les cochons, les volailles, le bétail, les poissons, les crustacés, etc.

Selon un autre mode de réalisation, on utilise un facteur d'appétence conforme à la présente invention en association avec un ou plusieurs autres facteurs d'appétence tels que des facteurs d'appétence d'origine animale et/ou végétale.

Selon encore un autre mode de réalisation, on utilise le facteur d'appétence objet de l'invention à raison de 1 à 10% en poids environ, de préférence à raison de 2 à 4 % en poids environ.

Un deuxième aspect de la présente invention vise un facteur d'appétence fermenté concentré susceptible d'être obtenu par concentration et/ou déshydratation d'au moins un facteur d'appétence tel que décrit supra.

Selon un troisième aspect, la présente invention concerne une composition de facteurs d'appétence destinée à être ajoutée à l'alimentation animale, comprenant au moins un facteur d'appétence comme mentionné ci-dessus. Une telle composition peut ne comprendre que des facteurs d'appétence selon la présente invention, ou bien comprendre également un ou plusieurs facteurs d'appétence d'origine animale et/ou végétale.

Dans un quatrième aspect, la présente invention a trait à un procédé de préparation d'un facteur d'appétence conforme à la description qui précède, ledit procédé comprenant au moins les étapes suivantes :
a) préculture d'au moins une levure telle que décrite ci-dessus dans un milieu de croissance standard, à une température allant de 22°C à 32°C environ, pendant 6 à 25 heures environ ;
b) ensemencement d'un milieu de fermentation contenant un ou plusieurs acides aminés, à l'aide de ladite préculture à un taux d'au moins 1.10⁵ cellules/ml environ ;
c) culture du milieu de fermentation ensemencé pendant 12 à 72 heures environ, à une température allant de 20°C à 32°C environ ;
d) arrêt de la fermentation ; et
e) récupération du milieu de fermentation final, utile comme facteur d'appétence.

Selon des modes de réalisation particuliers, dont il est éventuellement possible de combiner une ou plusieurs caractéristiques :
- l'étape de préculture a) est réalisée à une température de 30°C environ ;
- l'étape de préculture a) est maintenue pendant 8 à 16 heures environ ;
- l'étape de culture c) est maintenue pendant 12 à 60 heures environ, ou pendant 12 à 55 heures environ, ou encore pendant 20 à 72 heures environ, ou bien encore pendant 30 à 50 heures environ ;
- l'étape de culture c) est réalisée à une température allant de 28°C à 30°C environ.

En particulier, la préculture sera maintenue jusqu'à la fin de la phase exponentielle de croissance de la levure.

De manière avantageuse, le milieu de croissance standard utilisé pour la préculture comprend des extraits de levure, de malt et des sucres tels que le glucose. Il peut comprendre en outre de la peptone et/ou des oligoéléments. Par exemple, ce milieu peut être un milieu Yeast Malt (YM) connu de l'homme du métier.

Selon un mode de réalisation, le milieu de fermentation utilisé à l'étape b) comprend environ 2 à 10% en poids, de préférence environ 3 à 5% en poids, d'un ou plusieurs acides aminés. Ces acides aminés sont, de manière préférée, des acides aminés aptes à être métabolisés par la levure. Alternativement, parmi les acides aminés présents dans le milieu de fermentation, il peut y avoir des acides aminés aptes à être métabolisés par la levure et des acides aminés non métabolisés, apportés en tant que suppléments. Le cas échéant, ces acides aminés supplémentaires peuvent avoir un effet positif sur l'appétence du facteur obtenu, par exemple en améliorant ses propriétés organoleptiques. Les proportions des différents acides aminés peuvent varier, avec une préférence pour une prépondérance d'acides aminés aptes à être métabolisés. Ceux-ci sont notamment choisis parmi l'alanine, la thréonine, la lysine et les acides aminés soufrés. De préférence, ce ou ces acides aminés sont choisis parmi les acides aminés soufrés, tels que la méthionine et la cystéine. On préférera utiliser la méthionine.

Selon un autre mode de réalisation, le milieu de fermentation utilisé à l'étape b) comprend en outre jusqu'à 10% en poids environ, de préférence entre 5 et 10% en poids environ, de matières grasses.

Selon encore un autre mode de réalisation, le milieu de fermentation utilisé à l'étape b) comprend en outre des extraits de levure et de malt.

Selon des modes de réalisation préférés, l'ensemencement du milieu de fermentation à l'aide de la préculture (étape b) est effectué à un taux au moins égal à, par ordre croissant de préférence, 5.10⁵, 1.10⁶, 4.10⁶, 6.10⁶ cellules/ml environ, c'est-à-dire de manière à atteindre une population cellulaire en début de culture d'au moins 5.10⁵, 1.10⁶, 4.10⁶, 6.10⁶ cellules/ml environ dans le milieu de fermentation.

Selon un mode de réalisation, le pH du milieu de fermentation est ajusté au début de l'étape c) à une valeur allant de 4,5 à 6,5 environ, de préférence de 5,4 à 5,8 environ.

Selon encore un autre mode de réalisation, la préculture de l'étape a) est réalisée dans des conditions d'aération allant de 0,5 à 1,5 wm environ ; et/ou la culture de l'étape c) est réalisée dans des conditions d'aération allant de 0,02 à 0,40 wm environ.

Selon encore un autre mode de réalisation, la préculture de l'étape a) et/ou ladite culture de l'étape c) est(sont) maintenue(s) sous agitation.

Selon le procédé objet de l'invention, le facteur d'appétence étant constitué par le milieu de fermentation final, on pourra choisir de se débarrasser de la biomasse ; ou de garder le milieu de fermentation final comprenant la biomasse, que l'on veillera alors à inactiver. Ainsi, avant l'étape e), le milieu de fermentation peut être débarrassé de la biomasse, de préférence par une technique de séparation choisie parmi la filtration, la décantation, la centrifugation. Alternativement, les étapes d) à e), y compris l'étape d'élimination de la biomasse, pourront être réalisées simultanément, de préférence par filtration stérilisante. Ou bien, si la biomasse n'est pas éliminée, l'étape d) pourra par exemple être réalisée par inactivation thermique des levures.

Un cinquième aspect de la présente invention concerne un procédé d'amélioration de l'appétence de l'alimentation animale, qui comprend l'ajout à l'alimentation animale d'au moins un facteur d'appétence ou d'au moins une composition comme décrit plus haut.

En particulier, le facteur d'appétence ou la composition est ajouté(e) à l'alimentation animale seul(e) ou en combinaison avec un ou plusieurs autres facteurs d'appétence, tels que des facteurs d'appétence d'origine animale et/ou végétale.

De préférence, on utilise le facteur d'appétence ou la composition objet de l'invention à raison de 1 à 10% en poids environ, de préférence à raison de 2 à 4 % en poids environ.

Selon un sixième aspect, la présente invention vise l'utilisation d'au moins une levure pour préparer un facteur d'appétence comme susmentionné. De préférence, la levure est choisie parmi *Yarrowia sp., Saccharomyces sp., Candida sp., Kluyveromyces sp., Pichia sp., Debaryomyces sp., Zygosaccharomyces sp.* De préférence encore, la levure est choisie parmi *Yarrowia lipolytica, Saccharomyces cerevisiae, Candida versalitis, Kluyveromyces lactis, Kluyveromyces fragilis, Pichia pastoris, Debaryomyces harsenii, Zygosaccharomyces rouxii.* De manière préférée entre toutes, on utilise la levure *Yarrowia lipolytica.*

Selon un septième aspect, l'invention a pour objet l'utilisation d'un facteur d'appétence ou d'une composition conforme à l'invention, pour améliorer l'appétence de l'alimentation animale.

La présente invention divulgue également un aliment pour animaux comprenant au moins un facteur d'appétence ou au moins une composition comme décrit supra. En particulier, le facteur d'appétence ou la composition est appliqué(e) à et/ou incorporé(e) audit aliment, de préférence à raison de 1 à 10% en poids environ, de préférence encore à raison de 2 à 4 % en poids environ.

Les figures suivantes sont destinées à illustrer sans limiter la présente invention :

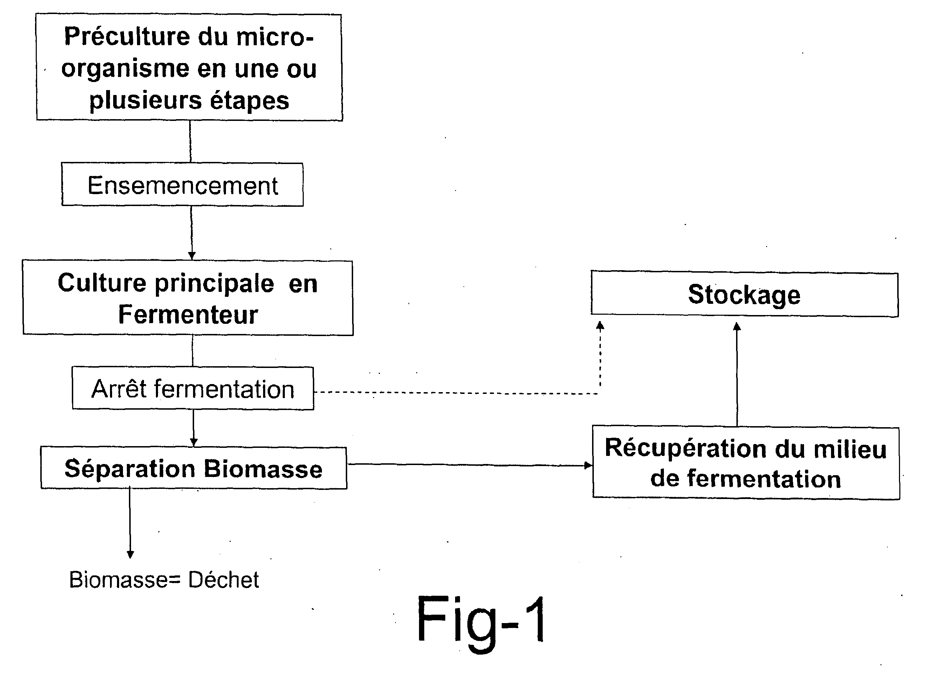

- figure 1 : schéma d'un exemple de procédé général de préparation d'un facteur d'appétence selon l'invention ;
- figure 2 : schéma d'un exemple de procédé de préparation d'un facteur d'appétence selon l'invention, en laboratoire (exemples 1 à 4) ;

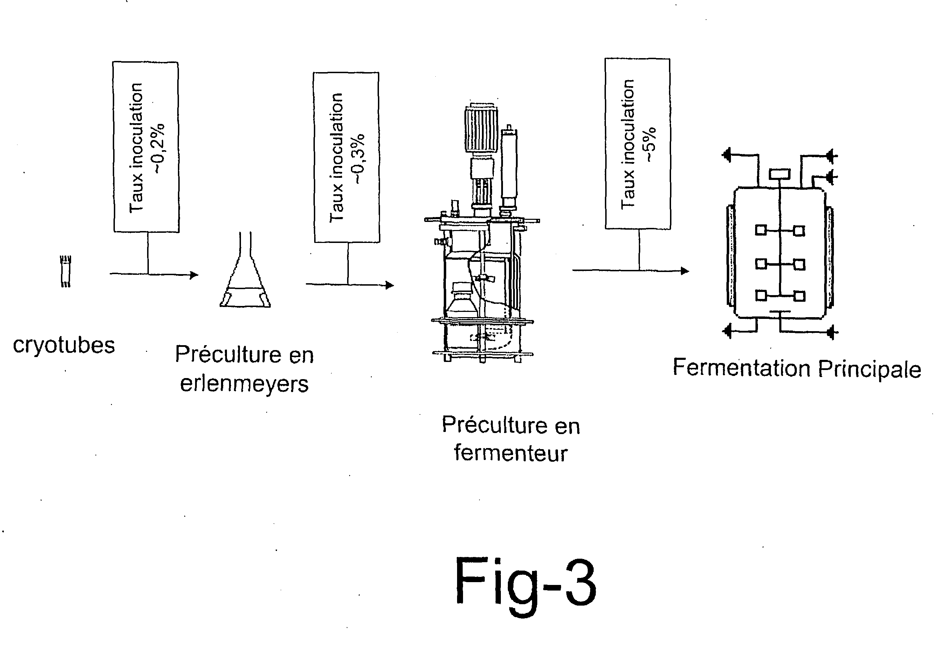

- figure 3 : schéma d'un exemple de procédé de préparation d'un facteur d'appétence selon l'invention, applicable à l'échelle industrielle (exemple 5) ;

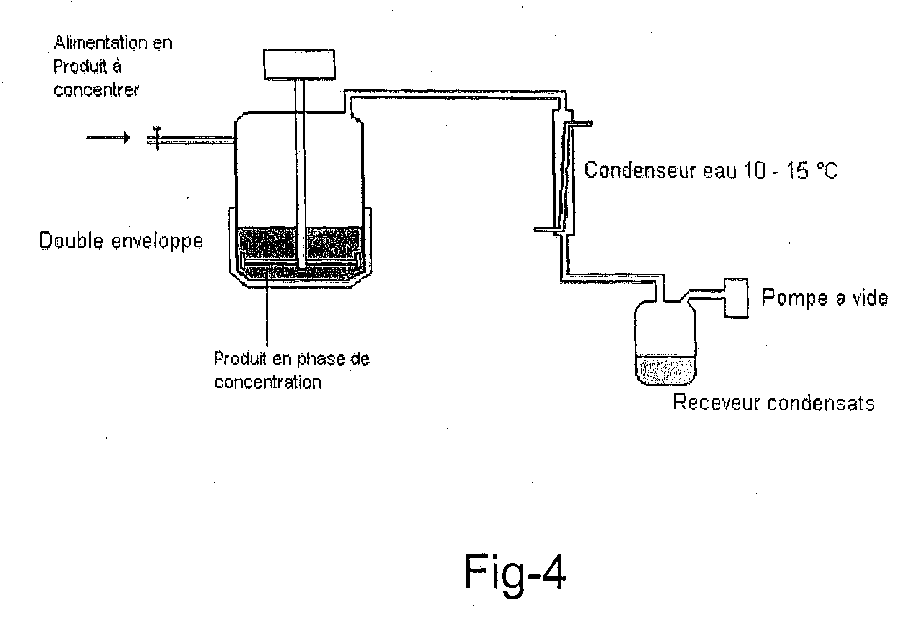

- figure 4 : schéma d'un exemple de procédé de concentration d'un facteur d'appétence selon l'invention (exemple 7) ;
- figure 5 : résultats d'un test d'appétence d'un facteur selon l'invention incorporé dans des aliments pour crevette (exemple 11). A : répartition des paniers dans le bassin ; B : résultats du test d'appétence.

Les exemples ci-dessous, donnés à titre purement indicatif, sont destinés à illustrer des modes de réalisation et avantages de la présente invention.

### EXEMPLES

### I- Exemples de facteurs d'appétence préparés à l'échelle du laboratoire

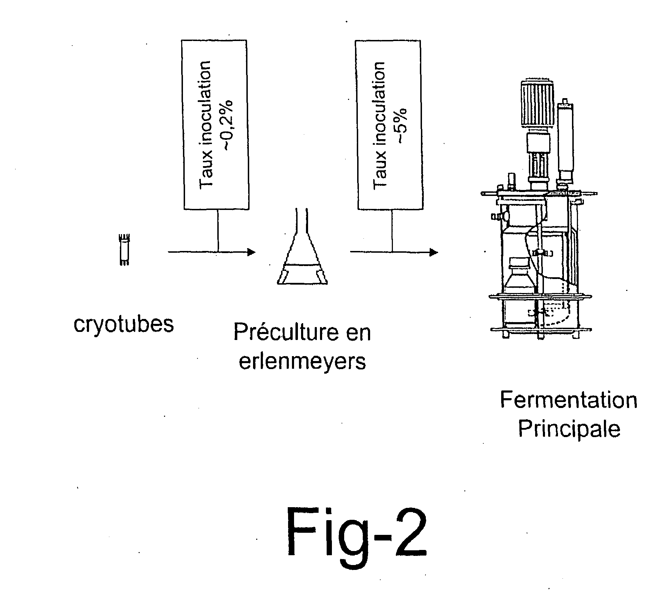

Un procédé général de préparation est schématisé sur la Fig.2.

### I-1- Exemple 1 : utilisation de la levure Yarrowia lipolytica et fermentation en présence de méthionine

La première étape était la préculture de *Yarrowia lipolytica* en erlenmeyers contenant 100 ml de milieu stérile de composition suivante : 0,25 gr d'extrait de malt (Muntons-UK), 0,2 gr d'extrait de levure (Biospringer - France), et 1,5 gr de glucose (Merck - Allemagne), qsp 100ml en eau déminéralisée. L'inoculation a été effectuée sous hotte stérile à partir de cryotubes de 1,8 ml, conservés à -80°C et issus de cultures sur milieu standard YM additionné de 50% de glycérol teneur 30%. La population théorique des cryotubes était de 6,5.10⁷ cellules/ml. Le pH a été ajusté en début de culture à 5,6 avec de l'acide chlorhydrique (Merck), les erlens placés sur table d'agitation Novotron (INFORS, Bottmingen, Suisse) à 30°C et à 140 tr/min. La préculture a été arrêtée après 16h d'incubation. En parallèle; on a préparé l'étape de fermentation principale. Pour cela, un fermenteur à cuve de verre 5L (Prélude- GUERIN SA- France) contenant 28 gr d'extrait de malt (Muntons- UK), 12 gr d'extrait de levure Biospringer - France), 3,4 gr d'H₃PO₄ 75% (Brenntag- France), 150 gr de Methionine (Adisseo - France), 5 ml d'antimousse (Struktol SB2020) et qsp 5 L d'eau déminéralisée, a été autoclavé à 121°C-15 min. En sortie d'autoclave, le fermenteur a été équipé d'un condenseur de sortie de gaz avec circuit d'eau glycolée à 2°C, et le milieu ramené à 30°C. L'aération a été fixée à 0,1 l/min grâce au débimètre massique sur l'alimentation du sparger de fond de cuve. L'agitation était de 500 tr/min, et la régulation de température fixée à 30°C. Le fermenteur a été inoculé stérilement avec la préculture (250 ml) de sorte que le population en début de fermentation était d'au moins 1.10⁵ cellules/ml. La fermentation a été conduite pendant 48h. Le fermenteur a été fermé hermétiquement avant d'être placé dans l'autoclave pour une inactivation à 110°C-10 min. Après refroisissement, le milieu de fermentation a été sorti du fermenteur. 30 gr de sorbate de potassium 50% (Nutrinova - Allemagne) puis 47 gr d'acide phosphorique à 75% y ont été ajoutés sous agitation. La composition finale du produit était la suivante : Humidité : 94,3%, Protéines (Nx6.25) Kjeldhal : 3,5%, MG par hydrolyse : <1,0%, Sucres solubles Totaux : 0,62%, Cendres : 1,64%, le pH était de 2,9.

### I-2- Exemple 2 : autre exemple d'utilisation de la levure Yarrowia lipolytica et de fermentation en présence de méthionine

Cet exemple a été réalisé dans les mêmes conditions que celles de l'exemple I-1, seule la composition du milieu pour la fermentation principale était différente. Celui-ci contenait 22 gr d'extrait de malt (Muntons- UK), 15 gr d'extrait de levure (Biospringer - France), 3,1 gr d'H₃PO₄ 75% (Brenntag-France), 150 gr de Methionine (Adisseo - France), 5 ml d'antimousse (Struktol SB2020), 180 gr de matière grasse laitière anhydre (Beuralia-France) et qsp 4 L d'eau déminéralisée.
La composition finale du produit était la suivante : humidité : 90,3%, protéines (Nx6,25) :3,4%, MG par hydrolyse :4,5%, sucres solubles totaux :0,7%, cendres :1,7. Le pH était de 2,9.

### I-3- Exemple 3 : utilisation de la levure Saccharomyces cerevisiae et fermentation en présence de méthionine

Cet exemple a été réalisé dans les mêmes conditions que celles de l'exemple 1, avec une préculture différente car la levure utilisée était *Saccharomyces cerevisiae.* Le milieu était constitué de 0,3 gr d'extrait de malt (Muntons-UK), 0,3 gr d'extrait de levure (Biospringer - France), 0,5 gr de peptone de viande (Merck Allemagne) et 12,5 gr de glucose (Merck - Allemagne). La préculture, dont le pH a été ajusté à 5,6, a été maintenue 13h à 30°C - 140 tr/min. La fermentation principale a été réalisée dans les mêmes conditions que dans l'exemple 1. La composition du produit fini était Humidité : 94,9%, Protéines (Nx6.25) Kjeldhal : 3,8%, MG par hydrolyse : <1,0%, Sucres solubles Totaux : 0,45%, Cendres : 1,3%, le pH était de 3.

### I-4- Exemple 4 : utilisation de la levure Zygosaccharomyces rouxii et fermentation en présence de cystéine

La levure Zygosaccharomyces *rouxii* a été cultivée sur milieu de préculture constitué de 0,3 gr d'extrait de malt (Muntons-UK), 0,3 gr d'extrait de levure (Biospringer - France), 0,5 gr de peptone de viande (Merck Allemagne) et 15 gr de glucose (Merck - Allemagne). La préculture, dont le pH a été ajusté à 5,6, a été maintenue 21 h à 30°C - 140 tr/min. La fermentation principale a été réalisée dans les mêmes conditions que dans l'exemple 1, mais la composition du milieu de fermentation était la suivante : 35 gr d'extrait de malt (Muntons- UK), 18 gr d'extrait de levure (Biospringer - France), 15,6 gr de NaOH 2M (Merck- Allemagne), 413 gr de Cysteine Monohydratée HCl (AMC-UK), 5 ml d'antimousse (Struktol SB2020) et qsp 5 L d'eau déminéralisée. La composition du produit fini était : Humidité : 88,2%, Protéines (Nx6.25) Kjeldhal : 7,1%, MG par hydrolyse : <1,0%, Sucres solubles Totaux : 0,83%, Cendres : 4,3%, le pH était de 2,9.

### II- Exemple 5 : facteur d'appétence préparé à l'échelle industrielle en utilisant la levure Yarrowia lipolytica, par fermentation en présence de méthionine

Le schéma de la Fig-3 illustre un exemple de procédé de préparation applicable à l'échelle industrielle.

La première étape était identique à celle de l'exemple 1. 15 ml de la préculture ont été prélevés stérilement et ont servi d'inoculum à un milieu stérile contenu dans un fermenteur de 5L (Prélude- GUERIN SA- France) contenant 15 gr d'extrait de malt (Muntons- UK), 15 gr d'extrait de levure (Biospringer - France), 50 gr de dextrose monohydraté (Tate & Lyle - Belgique), 12 gr d'H3PO4 75% (Brenntag- France, 5 ml d'antimousse (Struktol SB2020) et qsp 5 L d'eau déminéralisée. La température était de 30°C, l'aération a été fixée à 5 L/min, et l'agitation à 700 rpm. La fermentation a été conduite pendant 12h. Pendant ce temps, un fermenteur de capacité 60L (Fermenteur Semi-Automatique Pierre Guerin Biolafitte Type « S » 60/100L) a été équipé. Il contenait 300 gr d'extrait de malt, 180 gr d'extrait de levure, 152 gr d'acide phosphorique 75%, 2,4 kgs de méthionine, 80 gr d'antimousse Struktol et qsp d'eau déminéralisée pour un volume total de 60L. Le milieu a été stérilisé à 121°C pendant 20 minutes. Après refroisissement à 30°c, le pH a été ajusté à 5,7, et on a établi une pression de ciel de 0,5 bars, une aération de 24 L /min. Le condenseur de sortie d'air a été mis en route à la consigne 2°C. L'inoculation a été faite stérilement avec 3 litres d'inoculum directement issus du fermenteur de 5L à l'aide d'une pompe péristaltique (4L/h). Après 5 h de fermentation, l'aération a été abaissée à 6 L / min. La fermentation a été maintenue ainsi jusqu'à un temps total de 48h. L'inactivation a été faite à 110°C - 10 min dans le fermenteur. Une fois la température ramenée en dessous de 40°C, 360 gr de sorbate de Potassium à 50% et 542 gr d'acide Phosphorique 75% y ont été ajoutés. Le milieu de fermentation a été sorti du fermenteur et stocké en bidons de 25 kgs à température ambiante. La composition du produit était semblable à celle de l'exemple 1.

### III- Exemples de traitements particuliers

### III-1- Exemple 6 : illustration d'un procédé dans lequel on élimine la biomasse après fermentation

Le produit issu de l'exemple 1 a été traité de manière à le rendre exempt de microorganismes et à obtenir un produit parfaitement clair, translucide. Pour ce faire, un système de filtration fourni par PALL- France, support acétate de cellulose type K80, a été mis en oeuvre en sortie de fermenteur 100L. La surface du filtre était de 0,2 m². Le débit était de 300L /h/m². La rétention sur le filtre était de 0,2% en matière sèche. L'essai a donné lieu à un produit limpide, de composition suivante : Humidité: 95,8%, Protéines (Nx6.25) Kjeldhal : 3,05%, MG par hydrolyse : <1,0%, Sucres sol. Totaux : 0,41% et Cendres : 0,75%.

### III-2 Exemple 7 : préparation d'un facteur d'appétence concentré liquide

On a réalisé un produit appétent à plus forte matière sèche pour des commodités de stockage. 36 kgs du produit issu de l'exemple 1 ont été mis en oeuvre dans un procédé de concentration sous-vide schématisé sur la Fig-4. Le concentrateur était un concentrateur AURIOL, de capacité 50L, capacité utile 20L (alimentation en continu). La température de la double enveloppe a été fixée à 40°C, pour une température de produit de 35°C. Un réseau d'eau refroidie à 10°C assurait la condensation de l'eau évaporée. Le vide a été fixé au maximum (35 mbars). L'alimentation en produit a été effectuée manuellement, à une moyenne de 50L/h. La concentration a été arrêtée de manière à obtenir un produit concentré mais encore fluide. 4,5 kgs de produit ont été obtenus, de matière sèche égale à 27,5%. Le pH était de 3,3.

### III-3- Exemple 8 : préparation d'un facteur d'appétence concentré déshydraté

A partir du produit issu de l'exemple 1, une poudre a été réalisée sur matériel de marque Alfa-Laval type S18 et de capacité 20 à 40 kgs d'eau évaporée/h. 15 kgs de facteur d'appétence liquide ont été mixés à l'aide d'un ultraturax T50 pendant 5 minutes avec 6,4 kgs de levure de bière (Lorenzetti- Brésil). Le séchage d'une durée de 47 min, température entrée 165°C, température sortie poudre 90°C, a abouti à l'obtention de 4,4 kgs de poudre, avec une humidité de 6%.

### IV- Tests d'appétence d'un exemple de facteur selon l'invention

### IV-1- Exemple 9 : exemple d'un facteur appliqué par pulvérisation sur des aliments pour chien

L'application du facteur d'appétence issu de l'exemple 1 a été faite sous forme de pulvérisation du liquide, par « batch », à l'aide d'un enrobeur Forberg type RVC 120.
25 kgs de croquettes standard pour chien ont été placées à 25°C dans le Forberg. 1,662 kgs de graisse de saindoux chauffée à 60°C ont été pulvérisés par buse sur les croquettes pendant 50 secondes. Le mélange a été effectué pendant 1 minute. Puis 1,108 kgs de facteur d'appétence chauffé à 30°C ont été pulvérisés par buse, pendant 60 secondes. Le mélange a été maintenu encore 1 minute. Les croquettes enrobées ont été vidangées et conditionnées en sac pour être acheminées vers la structure de test. Une codification a été effectuée lors de la réalisation des batchs enrobage, et apparaissait sur le sac. Le produit A a été enrobé avec le facteur d'appétence issu de l'exemple 1. Le produit B était le témoin (enrobage avec un facteur d'appétence base carnée, issu de la gamme super-premium de la société SPF- France). Les conditions d'enrobage concernant le témoin étaient identiques à celles décrites pour l'obtention du produit A.

L'appétence de l'aliment ainsi obtenu a été mesurée au sein de la structure PANELIS - France, en panel expert, et selon une méthode de test versus. 2 repas ont été répartis sur la journée en panel de 36 chiens. 2 aliments ont été présentés à chaque animal simultanément, chaque aliment, noté A ou B, permettant de recouvrir les besoins alimentaires de chaque animal, et leur position étant inversée (droite ou gauche) d'un repas sur l'autre pour éviter un choix par latéralisation. On a relevé le 1^{er} aliment vers lequel l'animal se dirigeait (1^{er} choix) et la consommation finale de chaque aliment. Les résultats sont exprimés en % relatif de consommation de A ou B. Les résultats ont été traités statistiquement (Test Chi² pour le 1^{er} choix et test de student pour le ratio de consommation). Seuls les chiens « valides » ayant consommé normalement ont été comptabilisés. Les résultats récapitulés dans le tableau 1 montrent que les aliments enrobés avec le facteur d'appétence issu de l'exemple 1 sont préférés au témoin par les chiens.

**Tableau 1**

| **Test** | **1^{er} choix Test Chi²** | **Ratio consommation (%relatif)** | **Ratio Test student Seuil 5%** |
|---|---|---|---|
| **Repas 1** | | | |
| Produit A | 17,06*** | 84 | *** |
| Témoin - Produit B | | 16 | |
| **Repas 2** | | | |
| Produit A | 11,65 *** | 78 | *** |
| Témoin - Produit B | | 22 | |

| | | | |
|---|---|---|---|
| (***) : Très haute Significativité (p<0.01) | | | |

### IV-2- Exemple 10 : exemple d'un facteur d'appétence incorporé dans des aliments pour chien et chat

L'utilisation du facteur d'appétence issu de l'exemple 1 a été faite « en inclusion » dans une base standard pour aliment chien ou une base standard pour aliment chat au cours de l'extrusion à hauteur de 10% du poids total de la base alimentaire. L'extrusion a été réalisée à l'aide d'un extrudeur bivis corotatif Evolum 53 CLEXTRAL (France).
L'aliment A-CT était l'aliment chat contenant 10% du facteur d'appétence. Il était enrobé par de la graisse de volaille chauffée à 45°C à hauteur de 6% dans le Forberg type RVC 120, à 25°C. L'aliment A-CN était l'aliment chien contenant 10% du facteur d'appétence. Il était enrobé par de la graisse de saindoux chauffée à 60°C à hauteur de 6% dans le Forberg type RVC 120, à 25°C. Les aliments T-CT et T-CN étaient les aliments secs témoins sans inclusion de facteur d'appétence chat et chien respectivement, mais enrobés avec de la graisse comme spécifié ci-dessus. Une même quantité de ces aliments a été utilisée lors d'une deuxième série, mais cette fois-ci avec un enrobage extérieur d'un facteur d'appétence Super-Premium SPF à base de protéines animales, à 30°C, dans le Forberg type RVC 120, à 25°C, dans des conditions équivalentes à celles de l'exemple 9. Les lots de cette deuxième série ont été étiquetés Aliment A-CT-E, Aliment A-CN-E, Aliment T-CT-E et Aliment T-CN-E.
L'appétence des aliments ainsi obtenus a été mesurée au sein de la structure PANELIS - France, en panel expert, et selon une méthode de test versus. 2 repas ont été répartis sur la journée en panel de 36 chiens, ou sur 2 jours en panels de 40 chats. Les tests ont été réalisés et analysés dans les mêmes conditions que celles décrites dans l'exemple 9. Les résultats récapitulés dans le tableau 2 montrent que les aliments qui contiennent le facteur d'appétence issu de l'exemple 1 sont préférés aux témoins par les chiens et les chats.

**Tableau 2**

| **Panel** | **Test** | **1^{er} choix Test Chi²** | **Ratio consommation (% relatif)** | **Ratio Test student Seuil 5%** |
|---|---|---|---|---|
| **CHATTERIE** | **Repas 1** | | | |
| Série 1 | Aliment A-CT | nd | 59 | * |
| | Aliment T-CT | | 41 | |
| | **Repas 2** | | | |
| | Aliment A-CT | nd | 57 | * |
| | Aliment T-CT | | 43 | |
| **CHATTERIE** | **Repas 1** | | | |
| Série 2 | Aliment A-CT-E | nd | 68 | ** |
| | Aliment T-CT -E | | 31 | |
| | **Repas 2** | | | |
| | Aliment A-CT-E | nd | 61 | * |
| | Aliment T-CT -E | | 33 | |
| **CHENIL** | **Repas 1** | | | |
| Série 1 | Aliment A-CN | 16,20*** | 91 | *** |
| | Aliment T-CN | | 9 | |
| | **Repas 2** | | | |
| | Aliment A-CN | 9,14 ** | 84 | *** |
| | Aliment T-CN | | 16 | |
| **CHENIL** | **Repas 1** | | | |
| Série2 | Aliment A-CN-E | 5,45 * | 75 | ** |
| | Aliment T-CN-E | | 25 | |
| | **Repas 2** | | | |
| | Aliment A-CN-E | 4,80 * | 70 | * |
| | Aliment T-CN-E | | 30 | |

| | | | | |
|---|---|---|---|---|
| (***) : Très haute Significativité (p<0.01) (**) : Haute Significativité (p<0.05) (*) : Significativité (p<0.1) () : Non significatif | | | | |

### IV-3- Exemple 11 : exemple d'un facteur d'appétence incorporé dans des aliments pour crevette

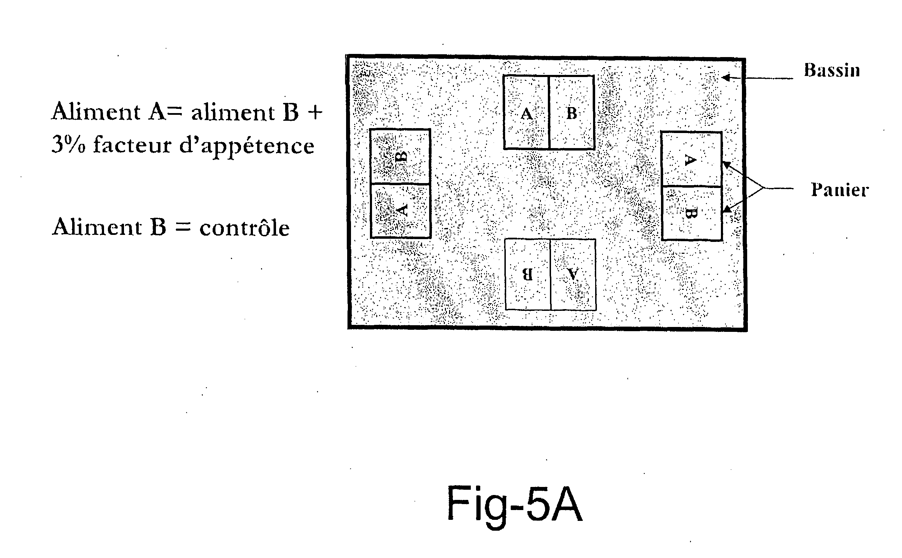

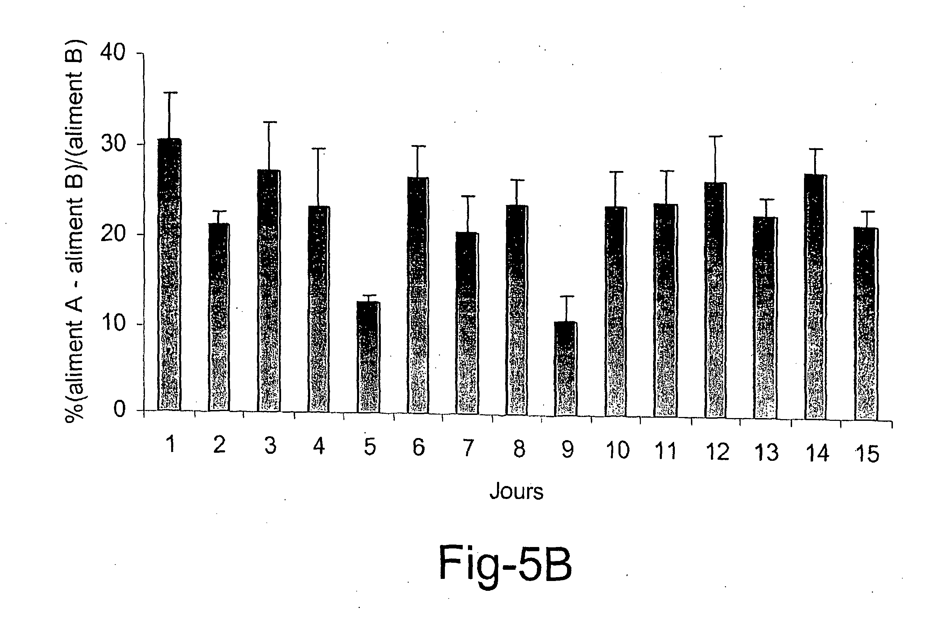

Le facteur d'appétence issu de l'exemple 1 a été incorporé en inclusion à hauteur de 3% dans un aliment de type commercial de formule suivante : farine de poissons 40%, blé entier 25%, remoulage de blé 15%, tourteaux de soja 9.5%, farine de crevettes 6%, huile de poissons 2%, lécithine de soja 1%, mélange vitaminique 1% et mélange minéral 0.5%. L'aliment obtenu (aliment A) par pressage sur presse Hobart (Allemagne) a été comparé à un aliment de formule identique mais sans facteur d'appétence (aliment B=contrôle).
Les essais se sont déroulés sur l'espèce crevette *P*. *vanamei* en ferme d'élevage (Brésil) dans un bassin de 1000m². Le poids moyen des crevettes était de 8g et la densité était de 20 animaux/m². Les crevettes ont été nourries à l'aide de 4 paniers disposés à des points précis dans le bassin. Chaque panier était séparé en deux parties sur lesquelles les aliments A et B prennaient place. La figure 5A illustre la répartition des paniers dans le bassin. Chaque aliment a été disposé dans les paniers à hauteur de 4% de la biomasse par jour et ce, en deux distributions réparties sur la journée. Les aliments étaient disposés sur les paniers en début de matinée et d'après-midi, et les paniers étaient relevés en fin de matinée et d'après-midi pour quantification de l'aliment non ingéré par les crevettes. Les opérations ont été renouvelées tous les jours pendant 15 jours.
Le paramètre suivant a été calculé pour chaque jour d'essai : [(Aliment A ingéré - Aliment B ingéré)/Aliment B ingéré]*100. Les résultats sont présentés sur la fig. 5B.
A la fin des 15 jours d'essai, la consommation de l'aliment A contenant le facteur d'appétence a été 23% plus élevée que celle de l'aliment B sans facteur d'appétence. L'aliment A contenant le facteur d'appétence a été significativement préféré à l'aliment B (P<0.05).

### V- Exemple 12 : composition de facteurs d'appétence

Un facteur d'appétence a été préparé en mélangeant le produit issu de l'exemple 1 avec un facteur d'appétence Super Premium à base de protéines animales. Les proportions des mélanges sont récapitulées dans le tableau 3. Les mélanges ont été réalisés à l'aide d'un mélangeur disperseur Ultraturax T50.

**Tableau 3**

| **Mélange n°** | **Proportion de facteur d'appétence exemple 1 (%)** | **Proportion Facteur d'appétence à base de protéines animales (%)** |
|---|---|---|
| **1** | 10 | 90 |
| **2** | 20 | 80 |
| **3** | 33 | 66 |
| **4** | 50 | 50 |

Des aliments pour chiens ont été réalisés par sprayage des mélanges sur base croquettes en Forberg type RVC 120. 24,955 kgs de croquettes standard pour chien ont été placées à 25°C dans le Forberg. 1,654 kgs de graisse de saindoux chauffée à 60°C ont été pulvérisés par buse sur les croquettes pendant 50 secondes. Le mélange a été effectué pendant 1 minute. Puis 831 grammes de facteur d'appétence chauffé à 30°C ont été pulvérisés par buse, pendant 60 secondes. Le mélange a été maintenu encore 1 minute. Les croquettes enrobées ont été vidangées et conditionnées en sac pour être acheminées vers la structure de test. Le témoin était l'aliment enrobé avec le facteur d'appétence base carnée seul ayant servi au mélange, issu de la gamme super-premium de la société SPF (France) et dans les mêmes conditions.
L'appétence des aliments ainsi obtenus a été mesurée au sein de la structure PANELIS - France, dans les conditions décrites dans l'exemple 9. Les résultats récapitulés dans le tableau 4 montrent que les facteurs d'appétence constitués des mélanges rendent les aliments sur lesquels ils sont pulvérisés plus appétents que les témoins.

**Tableau 4**

| **Test** | **Ratio consommation (%relatif)** | **Test student** Seuil 5% |
|---|---|---|
| **Repas 1** | | |
| Aliment Mélange 1 | 60 | ** |
| Témoin | 40 | |
| **Repas 2** | | |
| Aliment Mélange 1 | 58 | |
| Témoin | 41 | |
| | | |
| **Repas 1** | | |
| Aliment Mélange 2 | 68 | * |
| Témoin | 32 | |
| **Repas 2** | | |
| Aliment Mélange 2 | 72 | ** |
| Témoin | 41 | |
| | | |
| **Repas 1** | | |
| Aliment Mélange 3 | 75 | ** |
| Témoin | 25 | |
| **Repas 2** | | |
| Aliment Mélange 3 | 70 | |
| Témoin | 30 | ** |
| | | |
| **Repas 1** | | |
| Aliment Mélange 4 | 76 | *** |
| Témoin | 24 | |
| **Repas 2** | | |
| Aliment Mélange 4 | 75 | ** |
| Témoin | 25 | |

| | | |
|---|---|---|
| (***) : Très haute Significativité (p<0.01) (**) : Haute Significativité (p<0.05) (*) : Significativité (p<0.1) () : Non significatif | | |

## Revendications

1. Facteur d'appétence fermenté destiné à être ajouté à l'alimentation animale, **caractérisé en ce qu'**il comprend le milieu de fermentation obtenu après culture d'au moins une levure, ledit milieu de fermentation contenant au moins :
- humidité (H) : de 80 à 96% en poids environ ;
- fraction protéique (FP) : de 1,5 à 10,0% en poids environ ;
- matières grasses (MG) : jusqu'à 10% en poids environ ;
- sucres (S) : de 0,20 à 2,0% en poids environ ; et
- cendres (C) : de 0,3 à 8,0% en poids environ,
où ladite fraction protéique (FP) est dépourvue de constituants d'origine animale.

2. Facteur d'appétence selon la revendication 1, **caractérisé en ce que** lesdits constituants de ladite fraction protéique (FP) sont choisis parmi des protéines, des peptides, des acides aminés, et leurs combinaisons.

3. Facteur d'appétence selon la revendication 1 ou 2, **caractérisé en ce que** ladite fraction protéique (FP) comprend au moins 90% en poids environ d'un ou plusieurs acides aminés choisis parmi l'alanine, la thréonine, la lysine, les acides aminés soufrés, et sont de préférence choisis parmi les acides aminés soufrés.

4. Facteur d'appétence selon la revendication 3, **caractérisé en ce que** ladite fraction protéique (FP) comprend au moins 95%, de préférence au moins 98%, de préférence encore au moins 99% en poids environ du ou desdits acides aminés.

5. Facteur d'appétence selon la revendication 3 ou 4, **caractérisé en ce que** lesdits acides aminés soufrés sont choisis parmi la méthionine et la cystéine, et sont de préférence des méthionines.

6. Facteur d'appétence selon l'une quelconque des revendications 1 à 5, **caractérisé en ce qu'**il présente un pH allant de 1,6 à 3,5 environ, de préférence un pH allant de 2,6 à 3,2 environ.

7. Facteur d'appétence selon l'une quelconque des revendications 1 à 6, **caractérisé en ce que** ladite levure est choisie parmi *Yarrowia sp., Saccharomyces sp., Candida sp., Kluyveromyces sp., Pichia sp., Debaryomyces sp., Zygosaccharomyces sp.*

8. Facteur d'appétence selon la revendication 7, **caractérisé en ce que** ladite levure est choisie parmi *Yarrowia lipolytica, Saccharomyces cerevisiae, Candida versalitis, Kluyveromyces lactis, Kluyveromyces fragilis, Pichia pastoris, Debaryomyces harsenii, Zygosaccharomyces rouxii.*

9. Facteur d'appétence selon la revendication 8, **caractérisé en ce que** ladite levure est *Yarrowia lipolytica.*

10. Facteur d'appétence selon l'une quelconque des revendications 1 à 9, **caractérisé en ce qu'**il se présente sous une forme liquide prête à l'emploi.

11. Facteur d'appétence selon l'une quelconque des revendications 1 à 10, en tant que produit intermédiaire dans la préparation d'un facteur d'appétence fermenté concentré.

12. Facteur d'appétence selon l'une quelconque des revendications 1 à 11, **caractérisé en ce qu'**il est appliqué à et/ou incorporé dans ladite alimentation animale.

13. Facteur d'appétence selon la revendication 12, **caractérisé en ce qu'**il est utilisé en association avec un ou plusieurs autres facteurs d'appétence tels que des facteurs d'appétence d'origine animale et/ou végétale.

14. Facteur d'appétence selon la revendication 12 ou 13, **caractérisé en ce qu'**il est utilisé à raison de 1 à 10% en poids environ, de préférence à raison de 2 à 4 % en poids environ.

15. Facteur d'appétence selon l'une quelconque des revendications 1 à 14, **caractérisé en ce que** ladite alimentation animale est destinée aux animaux de compagnie.

16. Facteur d'appétence fermenté concentré susceptible d'être obtenu par concentration et/ou déshydratation d'au moins un facteur d'appétence selon l'une quelconque des revendications 1 à 15.

17. Composition de facteurs d'appétence destinée à être ajoutée à l'alimentation animale, **caractérisée en ce qu'**elle comprend au moins un facteur d'appétence selon l'une quelconque des revendications 1 à 16.

18. Procédé de préparation d'un facteur d'appétence selon l'une quelconque des revendications 1 à 16, comprenant au moins les étapes suivantes :
a) préculture d'au moins une levure en milieu de croissance standard, à une température allant de 22°C à 32°C environ, pendant 6 à 25 heures environ ;
b) ensemencement d'un milieu de fermentation contenant un ou plusieurs acides aminés, à l'aide de ladite préculture à un taux d'au moins 1.10⁵ cellules/ml environ ;
c) culture du milieu de fermentation ensemencé pendant 12 à 72 heures environ, à une température allant de 20°C à 32°C environ .;
d) arrêt de la fermentation ; et
e) récupération du milieu de fermentation final utile comme facteur d'appétence.

19. Procédé selon la revendication 18, **caractérisé en ce que** le milieu de fermentation utilisé à l'étape b) comprend environ 2 à 10% en poids, de préférence environ 3 à 5% en poids, d'un ou plusieurs acides aminés.

20. Procédé selon la revendication 18 ou 19, **caractérisé en ce que** le ou lesdits acides aminés sont choisis parmi l'alanine, la thréonine, la lysine, les acides aminés soufrés, et sont de préférence choisis parmi les acides aminés soufrés.

21. Procédé selon l'une quelconque des revendications 18 à 20, **caractérisé en ce que** l'étape d) est réalisée par inactivation thermique des levures.

22. Procédé selon l'une quelconque des revendications 18 à 21, **caractérisé en ce que**, avant l'étape e), ledit milieu de fermentation est débarrassé de la biomasse.

23. Procédé selon la revendication 22, **caractérisé en ce que** les étapes d) à e) sont réalisées simultanément, de préférence par filtration stérilisante.

24. Procédé d'amélioration de l'appétence de l'alimentation animale, comprenant l'ajout d'au moins un facteur d'appétence selon l'une quelconque des revendications 1 à 16, ou d'au moins une composition selon la revendication 17, à ladite alimentation animale.

25. Utilisation d'au moins une levure pour préparer un facteur d'appétence selon l'une quelconque des revendications 1 à 16.

26. Utilisation selon la revendication 25, **caractérisée en ce que** ladite levure est *Yarrowia lipolytica.*

## Claims

1. A fermented appetence factor to be added to animal feed, **characterised in that** it comprises the fermentation medium obtained after culture of at least one yeast, said fermentation medium containing at least:
- humidity (H): from 80 to 96% by weight approximately;
- protein factor (FP) : from 1.5 to 10.0% by weight approximately;
- fatty matter (MG): up to 10% by weight approximately;
- sugars (S): from 0.20 to 2.0% by weight approximately; and
- ashes (C): from 0.3 to 8.0% by weight approximately,
where said protein factor (FP) is devoid of constituents of animal origin.

2. The appetence factor according to claim 1, **characterised in that** said constituents of said protein factor (FP) are selected from proteins, peptides, amino acids, and their combinations.

3. The appetence factor according to claim 1 or 2, **characterised in that** said protein factor (FP) comprises at least 90% by weight approximately of one or more amino acids selected from alanine, threonine, lysine, sulphured amino acids, said are preferably selected from sulphured amino acids.

4. The appetence factor according to claim 3, **characterised in that** said protein factor (FP) comprises at least 95%, preferably at least 98%, preferably again at least 99% by weight approximately of said amino acids.

5. The appetence factor according to claim 3 or 4, **characterised in that** said sulphured amino acids are selected from methionine and cystein, and are preferably methionines.

6. The appetence factor according to any one of Claims 1 to 5, **characterised in that** it has a pH ranging from 1.6 to 3.5 approximately, preferably a pH ranging from 2.6 to 3.2 approximately.

7. The appetence factor according to any one of Claims 1 to 6, **characterised in that** said yeast is selected from *Yarrowia sp., Saccharomyces sp,, Candida sp., Kluyveromyces sp., Pichia sp., Debaryomyces sp., Zygosaccharomyces sp.*

8. The appetence factor according to claim 7, **characterised in that** said yeast is selected from *Yarrowia lipolytica, Saccharomyces cerevisiae, Candida versalitis, Kluyveromyces lactis, Kluyveromyces fragilis, Pichia pastoris, Debaryomyces harsenii, Zygosaccharomyces rouxii.*

9. The appetence factor according to claim 8, **characterised in that** said yeast is *Yarrowia lipolytica.*

10. The appetence factor according to any one of Claims 1 to 9, **characterised in that** it is in liquid form ready for use.

11. The appetence factor according to any one of Claims 1 to 10, as an intermediary product in the preparation of a concentrated fermented appetence factor.

12. The appetence factor according to any one of Claims 1 to 11, **characterised in that** it is applied to and/or incorporated in said animal feed.

13. The appetence factor according to claim 12, **characterised in that** it is utilised in association with one or more other appetence factors such as appetence factors of animal and/or vegetable origin.

14. The appetence factor according to claim 12 or 13, **characterised in that** it is utilised at a rate of 1 to 10% by weight approximately, preferably at a rate of 2 to 4 % by weight approximately.

15. The appetence factor according to any one of Claims 1 to 14, **characterised in that** said animal feed is intended for pets.

16. A concentrated fermented appetence factor to be obtained by concentration and/or dehydration of at least one appetence factor according to any one of Claims 1 to 15.

17. Composition of appetence factors to be added to animal feed, **characterised in that** it comprises at least one appetence factor according to any one of Claims 1 to 16.

18. A method of preparation of an appetence factor according to any one of claims 1 to 16, comprising at least the following steps:
a) preculture of at least one yeast in standard growth medium, at a temperature ranging from 22°C to 32°C approximately, for 6 to 25 hours approximately;
b) seeding of a fermentation medium containing one or more amino acids, by means of said preculture at a rate of at least 1.10⁵ cells/ml approximately;
c) culture of the seeded fermentation medium for 12 to 72 hours approximately, at a temperature ranging from 20°C to 32°C approximately;
d) termination of fermentation; and
e) recovery of the final fermentation useful medium as appetence factor.

19. The method according to claim 18, **characterised in that** the fermentation medium utilised in step b) comprises approximately 2 to 10% by weight, preferably approximately 3 to 5% by weight, of one or more amino acids.

20. The method according to claim 18 or 19, **characterised in that** said amino acids are selected from alanine, threonine, lysine, sulphured amino acids, and are preferably selected from sulphured amino acids.

21. The method according to any one of claims 18 to 20, **characterised in that** step d) is carried out by thermal inactivation of yeasts.

22. The method according to any one of claims 18 to 21, **characterised in that**, prior to step e), said fermentation medium is stripped of the biomass.

23. The method according to claim 22, **characterised in that** the steps d) to e) are carried out simultaneously, preferably by sterilising filtration.

24. A method for improving the appetence of animal feed, comprising adding at least one appetence factor according to any one of Claims 1 to 16, or at least one composition according to claim 17, to said animal feed.

25. Use of at least one yeast for preparing an appetence factor according to any one of Claims 1 to 16.

26. Use according to claim 25, **characterised in that** said yeast is *Yarrowia lipolytica.*

## Patentansprüche

1. Fermentierter Appetenzfaktor, welcher dazu bestimmt ist, zur Tiernahrung hinzugefügt zu werden, **dadurch gekennzeichnet, dass** er das Fermentationsmedium, das nach einer Kultivierung von mindestens einer Hefe erhalten wird, umfasst, wobei das Fermentationsmedium mindestens enthält:
- Feuchtigkeit (H): etwa 80 bis 96 Gew.-%;
- Proteinanteil (FP): etwa 1,5 bis 10,0 Gew.-%;
- Fette (MG): bis zu etwa 10 Gew.-%;
- Zucker (S): etwa 0,20 bis 2,0 Gew.-%; und
- Ascherückstände (C): etwa 0,3 bis 8,0 Gew.-%,
wobei der Proteinanteil (FP) frei ist von Bestandteilen tierischer Herkunft.

2. Appetenzfaktor nach Anspruch 1, **dadurch gekennzeichnet, dass** die Bestandteile des Proteinanteils (FP) unter Proteinen, Peptiden, Aminosäuren und deren Kombinationen ausgewählt sind.

3. Appetenzfaktor nach Anspruch 1 oder 2, **dadurch gekennzeichnet, dass** der Proteinanteil (FP) mindestens etwa 90 Gew.-% von einer oder mehreren Aminosäuren, die unter Alanin, Threonin, Lysin und den schwefelhaltigen Aminosäuren ausgewählt werden und vorzugsweise unter den schwefelhaltigen Aminosäuren ausgewählt werden, umfasst.

4. Appetenzfaktor nach Anspruch 3, **dadurch gekennzeichnet, dass** der Proteinanteil (FP) mindestens etwa 95 Gew.-%, vorzugsweise mindestens etwa 98 Gew.-%, noch mehr bevorzugt mindestens etwa 99 Gew.-% von der oder den Aminosäuren umfasst.

5. Appetenzfaktor nach Anspruch 3 oder 4, **dadurch gekennzeichnet, dass** die schwefelhaltigen Aminosäuren unter Methionin und Cystein ausgewählt sind und vorzugsweise Methionine sind.

6. Appetenzfaktor nach einem der Ansprüche 1 bis 5, **dadurch gekennzeichnet, dass** er einen pH, der von etwa 1,6 bis 3,5 geht, vorzugsweise einen pH, der von etwa 2,6 bis 3,2 geht, aufweist.

7. Appetenzfaktor nach einem der Ansprüche 1 bis 6, **dadurch gekennzeichnet, dass** die Hefe unter *Yarrowia sp., Saccharomyces sp., Candida sp., Kluyveromyces sp., Pichia sp., Debaryomyces sp., Zygosaccharomyces sp.* ausgewählt ist.

8. Appetenzfaktor nach Anspruch 7, **dadurch gekennzeichnet, dass** die Hefe unter *Yarrowia lipolytica, Saccharomyces cerevisiae, Candida versalitis, Kluyveromyces lactis, Kluyveromyces fragilis, Pichia pastoris, Debaryomyces harsenii, Zygosaccharomyces rouxii* ausgewählt ist.

9. Appetenzfaktor nach Anspruch 8, **dadurch gekennzeichnet, dass** die Hefe *Yarrowia lipolytica* ist.

10. Appetenzfaktor nach einem der Ansprüche 1 bis 9, **dadurch gekennzeichnet, dass** er in einer einsatzbereiten flüssigen Form vorliegt.

11. Appetenzfaktor nach einem der Ansprüche 1 bis 10 als Zwischenprodukt bei der Herstellung eines konzentrierten fermentierten Appetenzfaktors.

12. Appetenzfaktor nach einem der Ansprüche 1 bis 11, **dadurch gekennzeichnet, dass** er auf die Tiernahrung aufgebracht oder in diese eingearbeitet wird.

13. Appetenzfaktor nach Anspruch 12, **dadurch gekennzeichnet, dass** er in Kombination mit einem oder mehreren anderen Appetenzfaktoren, wie Appetenzfaktoren tierischer und/oder pflanzlicher Herkunft, eingesetzt wird.

14. Appetenzfaktor nach Anspruch 12 oder 13, **dadurch gekennzeichnet, dass** er in einer Menge von etwa 1 bis 10 Gew.-%, vorzugsweise in einer Menge von etwa 2 bis 4 Gew.-% eingesetzt wird.

15. Appetenzfaktor nach einem der Ansprüche 1 bis 14, **dadurch gekennzeichnet, dass** die Tiernahrung für Heim- oder Haustiere bestimmt ist.

16. Konzentrierter fermentierter Appetenzfaktor, der durch Aufkonzentrierung und/oder Dehydratisierung von mindestens einem Appetenzfaktor nach einem der Ansprüche 1 bis 15 erhalten werden kann.

17. Zusammensetzung von Appetenzfaktoren, welche dazu bestimmt ist, zur Tiernahrung hinzugefügt zu werden, **dadurch gekennzeichnet, dass** sie mindestens einen Appetenzfaktor nach einem der Ansprüche 1 bis 16 umfasst.

18. Verfahren zur Herstellung eines Appetenzfaktors nach einem der Ansprüche 1 bis 16, welches mindestens die folgenden Schritte umfasst:
a) Vorkultur von mindestens einer Hefe in einem Standard-Nährmedium bei einer Temperatur, die von etwa 22°C bis 32°C geht, während etwa 6 bis 25 Stunden;
b) Animpfen eines Fermentationsmediums, welches eine oder mehrere Aminosäuren enthält, mit Hilfe der Vorkultur in einer Konzentration von mindestens etwa 1.10⁵ Zellen/ml;
c) Kultur des angeimpften Fermentationsmediums während etwa 12 bis 72 Stunden bei einer Temperatur, die von etwa 20°C bis 32°C geht;
d) Stoppen der Fermentation; und
e) Gewinnung des endgültigen Fermentationsmediums, das als Appetenzfaktor nützlich ist.

19. Verfahren nach Anspruch 18, **dadurch gekennzeichnet, dass** das in dem Schritt b) eingesetzte Fermentationsmedium etwa 2 bis 10 Gew.-%, vorzugsweise etwa 3 bis 5 Gew.-% von einer oder mehreren Aminosäuren umfasst.

20. Verfahren nach Anspruch 18 oder 19, **dadurch gekennzeichnet, dass** die Aminosäure oder Aminosäuren unter Alanin, Threonin, Lysin und den schwefelhaltigen Aminosäuren ausgewählt werden und vorzugsweise unter den schwefelhaltigen Aminosäuren ausgewählt werden.

21. Verfahren nach einem der Ansprüche 18 bis 20, **dadurch gekennzeichnet, dass** der Schritt d) durch Hitzeinaktivierung der Hefen ausgeführt wird.

22. Verfahren nach einem der Ansprüche 18 bis 21, **dadurch gekennzeichnet, dass** vor dem Schritt e) das Fermentationsmedium von der Biomasse befreit wird.

23. Verfahren nach Anspruch 22, **dadurch gekennzeichnet, dass** die Schritte d) bis e) gleichzeitig, vorzugsweise durch sterilisierende Filtration, ausgeführt werden.

24. Verfahren zur Verbesserung der Appetenz von Tiernahrung, welches das Hinzufügen von mindestens einem Appetenzfaktor nach einem der Ansprüche 1 bis 16 oder von mindestens einer Zusammensetzung nach Anspruch 17 zu der Tiernahrung umfasst.

25. Verwendung von mindestens einer Hefe, um einen Appetenzfaktor nach einem der Ansprüche 1 bis 16 herzustellen.

26. Verwendung nach Anspruch 25, **dadurch gekennzeichnet, dass** die Hefe *Yarrowia lipolytica* ist.
